# EUROPEAN PATENT APPLICATION

(11) **EP 1 797 870 A1**
(43) Date of publication of application: **20.06.2007**
(21) Application number: 05027314.3
(22) Date of filing: 14.12.2005
(51) Int. Cl.: A61K 9/107, A61K 47/48

(54) **Oral delivery of protein drugs using microemulsion**

(71) Applicant: The Jordanian Pharmaceutical Manufacturing Co., 11710 Naor (JO)
(72) Inventor: Badwan, Adnan Ali, Naor 11710 (JO); Al-Remawi, Mayyas, Naor 11710 (JO); El-Thaher, Talal, Naor 11710 (JO); Elsayed, Amani, Naor 11710 (JO)
(74) Representative: Winkler, Andreas Fritz Ernst

(57) **Abstract**

A novel method of encapsulating a drug, preferably a peptide and/or protein drug in a biodegradable complex, which comprises: a) a biodegradable polymer that contains primary amine groups such as chitosan oligosaccharide, b) an organic carboxylic acid such as oleic acid, and c) optionally, an emulsifying agent such as plurol (glycerol-6-dioleate). The formula results in the formation of a continuous oily phase containing nanoparticles having the drug encapsulated therein.

## Description

### Field of the invention

The present invention is within the field of protection and oral delivery for drugs sensitive to acid and enzymes in the human gut, specifically peptide and protein drugs. However, this delivery system is also suitable for drugs suffering from first pass effect either due to rapid gastrointestinal or liver metabolism. More precisely the invention relates to a novel encapsulating method for manufacturing a formulation which is suitable for oral delivery of proteins for example insulin.

### Background

Since the discovery of insulin, diabetics have to inject their insulin doses subcutaneously. Oral insulin would present a more convenient form of application as pain caused by the injection, stress of multiple daily injections and possible infections could be avoided leading to a higher patient compliance. However, the oral delivery of many therapeutic peptides and proteins remain an unresolved challenge mainly because of the large size, hydrophilicity and instability of these macromolecules (Lee, V. H.L., Yamamoto, A., 1990. Adv.Drug Deliv.Rev., 4, 171-207). Many different strategies have been applied to develop a bioactive oral insulin formulation such as chemical modification of insulin, addition of enhancers and/or protease inhibitors, enteric coatings, use of carriers systems such as liposomes, mixed micelles, multiple emulsions, microemulsions, bioadhesive microparticles or nanoparticles (Y. Pan, Ying-Jian Li, Hui-ying Zhao,Jn-min Zheng, Hui Xu, Gang Wei, Jin-song Hao, Fu-de Cui, 2002. Int. J.Pharm., 249, 139-147). Similar problems apply to other drugs, in particular peptide or protein drugs.

Chitosan is an abundant natural polymer, obtained by alkaline N- deacetylation of chitin. Chitosan acts as an absorption enhancer in the intestine by increasing the residence time of dosage forms at mucosal sites, inhibiting proteolytic enzymes, and increasing the permeability of protein and peptide drugs across mucosal membranes (Hejazi R., and Amiji M., 2003., J. Controlled Release, 89 151-165). Chitosan-insulin nanoparticles prepared by iontotropic gelation were effective in lowering the serum glucose level of streptozotocin-induced diabetic rats when given at insulin doses of 50 and/ or 100 U/kg to the rats ( Ma Zengshuan, Lim T., M., and Lim Lee-Young, 2005. Int. J. Pharm., 293, 271-280). Chitosan nanoparticles, chitosan nanocapsules and chitosan-coated lipid nanoparticles were investigated as carriers for nasal/oral administration of macromolecules. Studies on the Caco-2 model cell line showed that the presence of chitosan on the nanosystems provides a modification of the transepithelial resistance and enhances their uptake (Prego C., Garcia M., Torres D., and Alonso M.J., 2005. J. Controlled Release, 101, 151-162).

While investigating methods to overcome barriers for oral protein drug delivery, surprisingly we found that complexing a drug such as insulin with a biodegradable polymer such as chitosan and incorporating the complex into a microemulsion will result in a dosage form suitable for oral drug delivery.

### Brief Description of the Invention

While trying to overcome barriers hindering delivery of protein and peptides through oral route, surprisingly we are able to do so by forming a complex between the peptide or protein drug for example insulin and chitosan of different molecular weights, the complex is formulated into a microemulsion, most preferable with nanosize. The microemulsion is made with emulsifying agents while fatty acids such as oleic acid are the continuous phase. The resulted product is suitable to be delivered orally.

### Examples

### Example 1

### Physical appearance showing the interaction of chitosan with free fatty acid

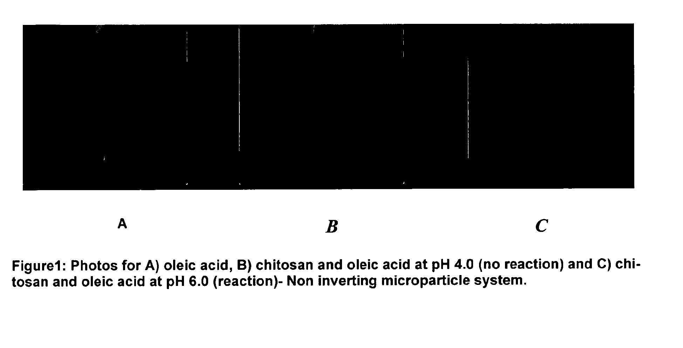

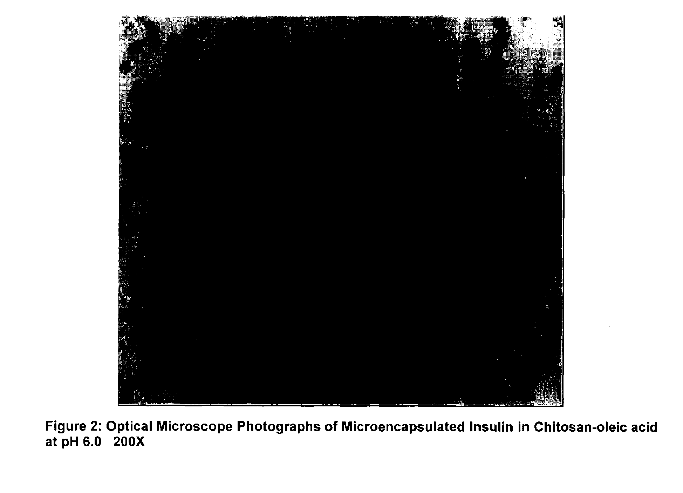

Chitosan oligosaccharide HCl when dissolved in water results in a pH of 4.0. while chitosan oligosaccharide base gives a pH of 6.0. Each aqueous system was mixed with oleic acid as an example of a fatty acid. Chitosan base reacted spontaneously with the free fatty acid forming a non-inverting w/o microparticulate system as shown in Fig 1 and 2. This rigid w/o system was non-inverting irrespective of the amount of aqueous solvent added to this system. A nano system was also possible by addition of surfactants such as plurol.

### Example 2

### In-vivo study on streptozotocin diabetic rat (fast state)

### Sample preparation

Chitosan < 5000 was dissolved in 0.1 M HCl. Recombinant human (rh)-insulin powder was weighed accurately and dissolved in chitosan solution. The pH of insulin-chitosan solution was adjusted to 7.3 with NaOH. 0.25 g of plurol (polyglyceryl-6-dioleate) and 0.25 g of labrasol (PEG 8 caprylic / capric glycerides) were mixed for 3 min. using a vortex mixer and left for 24 hrs to equilibrate. 2 g of oleic acid was added to the surfactants mixture and vortexed for 2 min. The obtained mixture was slowly titrated with the insulin-chitosan solution.

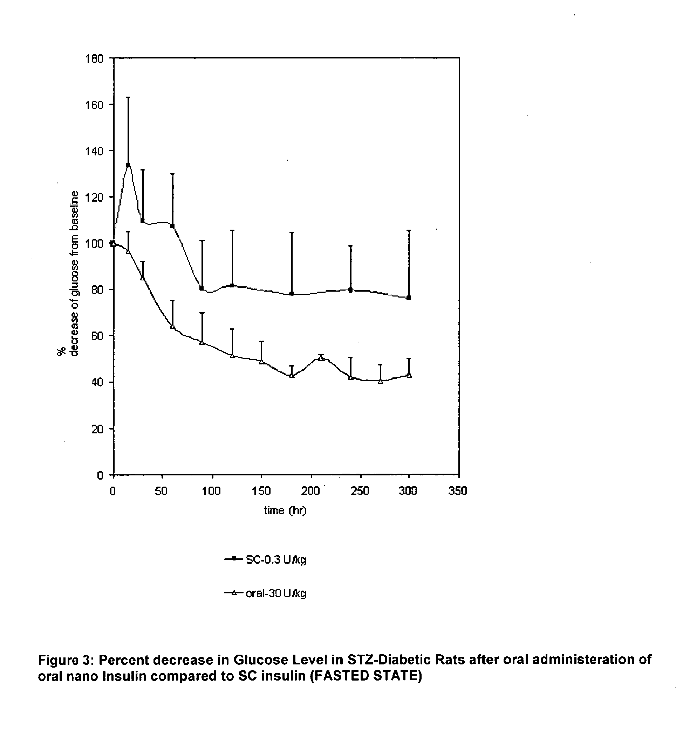

Male Wistar rats (weighing 200-300 g) were made diabetics by two intraperitoneal injections of streptozotocin dissolved in citrate buffer pH 4.5. They were considered to be diabetic when the base line glucose levels were above 250 mg/dl. The diabetic rats were fasted overnight and during the experiment but had free access to water. Animals were divided in two groups. Group 1 was given rh-insulin solution (0.3 IU /kg) subcutaneous. The other group was given our formula by oral gavage. Blood samples were obtained from tail vein before and after administration. Glucose levels were determined by a glucose reader. Plasma glucose levels after insulin administration were expressed as the percentage of the initial level. The data show the pharmacological effect of oral nanoencapsulated insulin in decreasing glucose level in diabetic rats, Fig. 3, thus, suggesting oral absorption of the protein drug.

### Example 3:

### In-vivo study on streptozotocin diabetic rat (fed state)

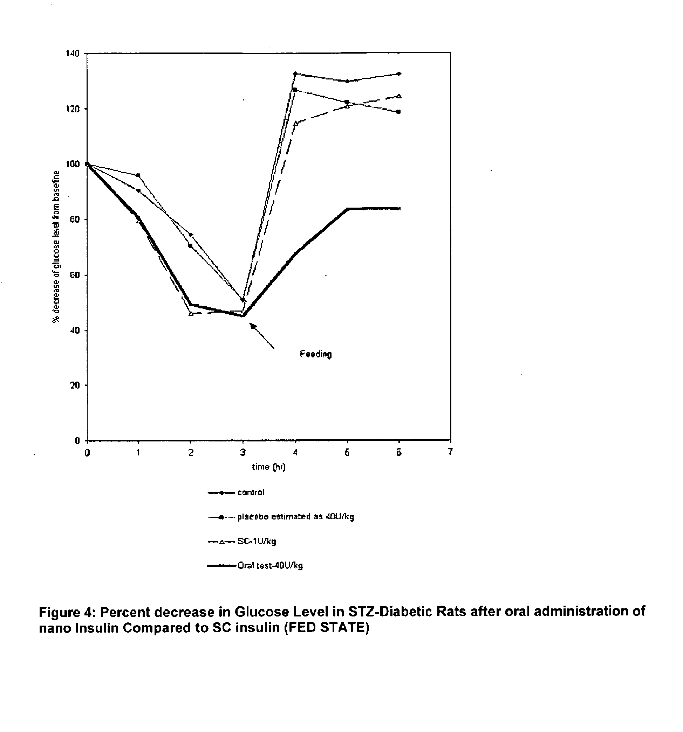

The same procedure were followed as in example 2 except that in this experiment rats were fed after 3 hours post administration of insulin oral dose. The results are shown in Fig 4. The results also provide a proof for the oral absorption of a protein drug i.e. insulin and suggesting a sustained action for ther oral nanoencaplsulated insulin system of the invention even after a meal was given to the rats.

## Claims

1. Method for encapsulating at least one drug for preparing a medicament for oral delivery of such drug, wherein a complex between the drug and a biodegradable polymer having primary amine groups is formulated into a w/o microemulsion wherein the continuous phase comprises at least one carboxylic acid.

2. Method according to claim 1, wherein the biodegradable polymer is chitosan or a chitosan derivative.

3. Method according to claim 1 or 2, wherein the organic acid is a fatty acid.

4. Method according to claim 3, wherein the fatty acid is oleic acid.

5. Method according to any of claims 1 to 4, wherein the microemulsion further comprises at least one emulsifying agent.

6. Method according to claim 5, wherein the emulsifying agent comprises at least one surfactant.

7. Method according to claim 6, wherein the surfactant comprises glycerol-6-dioleate.

8. Method according to any of the preceding claims, wherein the drug is a peptide or protein drug.

9. Method according to claim 8, wherein the drug is insulin.

10. Encapsulated drug producible according to any of the preceding claims.
